# EUROPEAN PATENT SPECIFICATION

(11) **EP 4 053 100 B1**
(45) Date of publication and mention of the grant of the patent: **28.05.2025**
(21) Application number: 20888486.6
(22) Date of filing: 13.11.2020
(51) Int. Cl.: C07C 68/06, C07C 68/08, B01D 3/00, B01D 3/14

(54) **METHOD FOR PREPARING BATTERY-GRADE METHYL ETHYL CARBONATE**
VERFAHREN ZUR HERSTELLUNG VON METHYLETHYLCARBONAT IN BATTERIEQUALITÄT
PROCÉDÉ DE PRÉPARATION DE CARBONATE D'ÉTHYLE MÉTHYLE DE QUALITÉ POUR BATTERIE

(30) Priority: 15.11.2019 CN 201911121000
(43) Date of publication of application: 07.09.2022
(73) Proprietor: Shida Shinghwa Advanced Material Group Co., Ltd., Dongying, Shandong 257503 (CN)
(72) Inventor: LI, Xin, Dongying, Shandong 257503 (CN); GUO, Jianjun, Dongying, Shandong 257503 (CN); MA, Guo, Dongying, Shandong 257503 (CN); LI, Guangke, Dongying, Shandong 257503 (CN)
(74) Representative: Bayramoglu et al.
(86) International application number: PCT/CN2020/128696
(87) International publication number: WO 2021/093854

(56) References cited:
- EP-B1- 2 679 572
- CN-A- 101 357 890
- CN-A- 101 723 834
- CN-A- 103 626 656
- CN-A- 105 439 857
- CN-A- 106 699 564
- CN-A- 106 699 565
- CN-A- 106 699 565
- CN-A- 106 748 792
- CN-A- 108 067 012
- CN-A- 109 535 000
- CN-A- 110 845 334
- CN-U- 206 886 993
- US-A1- 2014 081 039

## Description

### FIELD

The present application relates to a device and a method for preparing ethyl methyl carbonate, which belongs to the field of chemical industry.

### BACKGROUND

Ethyl Methyl Carbonate is a colorless and transparent liquid, insoluble in water, and can be used in organic synthesis. It is an excellent solvent for lithium-ion battery electrolyte.

The routes for synthetizing ethyl methyl carbonate by transesterification are as follows: (1) Dimethyl carbonate (DMC) reacts with anhydrous alcohol (EA) to generate ethyl methyl carbonate (EMC) and by product methanol, wherein the catalyst can be alkaline catalyst such as ionic liquid, alcohol-alkali or immobilized ionic liquid and alcohol-alkali; (2) Diethyl carbonate (DEC) reacts with methanol to generate ethyl methyl carbonate and by-product ethanol, wherein the catalyst is homogeneous or heterogeneous alkaline substance; (3) Dimethyl carbonate and diethyl carbonate generates ethyl methyl carbonate by transesterification, and no by product is generated, wherein the catalyst is alkaline substance. This route theoretically does not generate lower alcohols such as methanol and ethanol, but dimethyl carbonate and diethyl carbonate can still decompose under alkaline conditions to produce lower alcohols, which will affect the product content.

In the prior art, a homogeneous catalyst such as sodium methoxide or potassium methoxide is used and consumed in a large amount, which occupies heavy catalyst cost. In the prior art, each rectifying column top uses circulating water to extract heat and condense the steam of the column top, and the column bottom uses low pressure steam to supply heat to generate the rising material vapor required by the rectifying column, and each rectifying column top consumes cooling energy while the column bottom consumes heat. To this end, the present invention is specially proposed to reduce energy consumption and raw material production cost.

The quality of ethyl methyl carbonate used in lithium-ion battery electrolyte needs to meet the battery-grade requirement, and its quality indicators are: methanol content ≤ 0.0020%, ethanol content ≤ 0.0020%, ethyl methyl carbonate content ≥ 99.95%, and moisture content ≤ 0.0020 %. In practical applications, users of lithium ion electrolyte also require that ethyl methyl carbonate and lithium hexafluorophosphate be prepared into a solution according to a certain proportion and placed in an oven for heating chromaticity stability test. 20% lithium hexafluorophosphate solution is required to be baked in the oven at 60°C for 8 hours and the resulting chromaticity is not greater than 60.

CN 106748792 A discloses a method and an apparatus of producing methyl ethyl carbonate by azeotropic reaction rectification method, the apparatus includes an azeotropic reactive rectifying column, a dispensing tank, a methanol purification tower, and a dividing wall column. The apparatus allows a reaction, a rectification, and an azeotrope separation to be integrated in the azeotropic reactive rectifying column, and a ternary mixture of dimethyl carbonate, diethyl carbonate, and methyl ethyl carbonate can be separated using the dividing wall column, saving energy consumption, greatly simplifying process procedure, and saving equipment investment.

CN 103626656 A provides a method and an apparatus for separating dimethyl carbonate and methanol through heat pump pressure-swing rectification. In the method, an atmospheric azeotropic steam material obtained from the top of an atmospheric rectifying column is pressurized and heated by a heat pump compressor, and then introduced to a reboiler at the bottom of the atmospheric rectifying column as a heat source, so the liquid in the atmospheric rectifying column is heated, and meanwhile the self-condensation of the atmospheric azeotropic steam material is completed.

CN 108067012 A provides an extractant for separating methanol and dimethyl carbonate azeotrope by extractive rectification and an application and a processing method thereof. The extractant is a mixed extractant for including ionic liquid, the system includes a reactive rectifying column, an extractive rectifying column, and an extractant regeneration column that are sequentially connected. The extractive rectifying column carries out extractive rectification using above-mentioned extractant.

EP 2679572 B1 provides a method and an apparatus for the production of diaryl carbonate optionally integrated with a plant for the production of dialkyl carbonate via oxidative carbonylation of an alkyl alcohol, and especially to a method and an apparatus for the production of diphenyl carbonate (also referred to as "DPC") integrated with the production of dimethyl carbonate ("DMC") as starting raw material for the production of said diphenyl carbonate.

### SUMMARY

According to one aspect of the present application, there is provided a method for preparing battery-grade ethyl methyl carbonate, which is carried out by using at least one device of:
device I comprising a reactive rectifying column reboiler, a first compressor, a removal column, a product rectifying column, a second compressor, and a product rectifying column reboiler;
   a top of the removal column is connected with the reactive rectifying column reboiler through the first compressor so that a gas stream from the top of the removal column is allowed to enter the reactive rectifying column reboiler;
   the reactive rectifying column reboiler is connected with the removal column and the product rectifying column respectively so that the gas stream in the reactive rectifying column reboiler is allowed to enter the removal column and the product rectifying column;
   a top of the product rectifying column is connected with the product rectifying column reboiler through the second compressor so that the gas stream from the top of the product rectifying column is allowed to enter the product rectifying column reboiler; and
   the product rectifying column reboiler is connected with the top of the product rectifying column so that the gas stream in the product rectifying column reboiler is allowed to enter the product rectifying column;
   the device further comprises a first water cooler, the reactive rectifying column reboiler is connected with the removal column through the first water cooler; and the reactive rectifying column reboiler is connected with the product rectifying column through the first water cooler;
      and
   device II comprising a reactive rectifying column reboiler, a first compressor, a removal column, a product rectifying column, a second compressor, and a product rectifying column reboiler;
   a top of the removal column is connected with the reactive rectifying column reboiler through the first compressor so that a gas stream from the top of the removal column is allowed to enter the reactive rectifying column reboiler;
   the reactive rectifying column reboiler is connected with the removal column and the product rectifying column respectively so that the gas stream in the reactive rectifying column reboiler is allowed to enter the removal column and the product rectifying column;
   a top of the product rectifying column is connected with the product rectifying column reboiler through the second compressor so that the gas stream from the top of the product rectifying column is allowed to enter the product rectifying column reboiler; and
   the product rectifying column reboiler is connected with the top of the product rectifying column so that the gas stream in the product rectifying column reboiler is allowed to enter the product rectifying column;
   the device further comprises a second water cooler and the product rectifying column reboiler is connected with the top of the product rectifying column through the second water cooler;
the method comprises following steps:
   (1) passing the material comprising dimethyl carbonate and ethanol into a reactive rectifying column filled with a catalyst to perform a reaction in the reactive rectifying column, and extracting azeotrope of dimethyl carbonate and ethanol by a gas phase stream-extracting port of a top of the reactive rectifying column, and making one part of a mixture I comprising ethyl methyl carbonate, diethyl carbonate, dimethyl carbonate and ethanol enter the reactive rectifying column reboiler and the other part of the mixture I enter a feeding port of the removal column;
   (2) extracting a mixed steam of ethyl methyl carbonate and dimethyl carbonate from a gas phase stream-extracting port of the top of the removal column which is then subject to compression by the first compressor and is passed into the reactive rectifying column reboiler to supply heat to the reactive rectifying column reboiler, wherein a first part of the material entering the reactive rectifying column reboiler is returned to a bottom of the reactive rectifying column, and a second part is passed into a reflux port of the top of the removal column, and the remaining part is passed into a feeding port of the product rectifying column; and
   (3) extracting the mixed steam of ethyl methyl carbonate and dimethyl carbonate from a gas phase stream-extracting port of the top of the product rectifying column which is then subject to compression by the second compressor and is passed into the product rectifying column reboiler to supply heat to the product rectifying column reboiler, wherein one part of a mixture II comprising diethyl carbonate and ethyl methyl carbonate is passed into the product rectifying column reboiler from the bottom of the product rectifying column and the other part is passed into the feeding port of the removal column; a first part of the material entering the product rectifying column reboiler is returned to a bottom of the product rectifying column, a second part is returned to a reflux port of the top of the product rectifying column, and the remaining part as raw material is passed into the material comprising dimethyl carbonate and ethanol; and the battery-grade ethyl methyl carbonate can be extracted from a middle of the product rectifying column;
the azeotrope of dimethyl carbonate and ethanol is extracted from the gas phase stream-extracting port of the top of the reactive rectifying column, then enters into a first preheater where the azeotrope exchanges heat with the material comprising dimethyl carbonate and ethanol, and further then one part of the azeotrope is passed into a reflux port of the top of the reactive rectifying column and the other part is extracted and passed into a separation system for removing the azeotrope;
the method comprises passing the material containing dimethyl carbonate and ethanol into a static mixer, then passing the material through the first preheater, a second preheater and a third preheater in sequence, and entering the material into a pre-reactor filled with the catalyst to perform a pre-reaction in the pre-reactor followed by entering a resulting material into a feeding port of the reactive rectifying column.

Optionally, the catalyst is selected from alkaline catalysts.

Preferably, the catalyst comprises a carrier and a metal element, and the metal element is supported on the carrier; the metal element is selected from alkali metals; and the carrier is at least one of aluminum oxide, silicon oxide and molecular sieve.

Optionally, when the device I is used, after the temperature of the material in the reactive rectifying column reboiler is adjusted by the first water cooler, one part thereof flows back into the reflux port of the removal column, and the other part thereof is passed into the feeding port of the product rectifying column.

Optionally, when the device II is used, after the temperature of the material in the product rectifying column reboiler is adjusted by the second water cooler, one part thereof flows back to the reflux port of the product rectifying column and the other part as raw material is passed into the material containing dimethyl carbonate and ethanol.

Optionally, the battery-grade ethyl methyl carbonate extracted from the middle of the product rectifying column is passed into the second preheater for cooling and then extracted as a target product.

Optionally, one part of the material comprising diethyl carbonate and ethyl methyl carbonate extracted from a bottom of the removal column is passed into a removal column reboiler, and the other part is passed into a rectifying system for removing diethyl carbonate; one part of the material entering into the removal column reboiler is returned to the bottom of the removal column, and the other part is passed into the third preheater; and the removal column reboiler is supplied heat by steam.

Optionally, process operating conditions of the reactive rectifying column are as follows: a temperature of the top of the reactive rectifying column ranges from 60 to 70°C, a pressure of the bottom of the reactive rectifying column ranges from 100 to 150 KPa, a temperature of the bottom of the reactive rectifying column ranges from 80 to 120 °C, a pressure of the bottom of the reactive rectifying column ranges from 110 to 200 KPa and a reflux ratio of the top of the reactive rectifying column ranges from 2:1 to 8:1;
process operating conditions of the removal column are as follows: aa temperature of the top of the removal column ranges from 80 to 120 °C, a pressure of the top of the removal column ranges from 100 to 150 KPa, a temperature of a bottom of the removal column ranges from 120 to 140°C, a pressure of the bottom of the removal column ranges from 120 to 200 KPa, and a reflux ratio of the top of the removal column ranges from 1:1 to 6:1;
process operating conditions of the product rectifying column are as follows: a temperature of the top of the product rectifying column ranges from 90 to 120 °C, a pressure of the top of the product rectifying column ranges from 100 to 150 KPa, a temperature of the bottom of the product rectifying column ranges from 100 to 130°C, a pressure of the bottom of the product rectifying column ranges from 120 to 200 KPa, and a reflux ratio of the top of the product rectifying column ranges from 2:1 to 8:1; and
a reaction temperature of the pre-reactor ranges from 90 to 120°C.

Optionally, in the material comprising dimethyl carbonate and ethanol, a molar ratio of the dimethyl carbonate to the ethanol ranges from 0.5:1 to 2:1.

Optionally, the compression ratio of the first compressor ranges from 2 to 3, an outlet pressure of the first compressor ranges from 200 to 300 KPa, and an outlet temperature of the first compressor ranges from 120 to 145°C; and a compression ratio of the second compressor ranges from 2.0 to 3.5; an outlet pressure of the second compressor ranges from 200 to 360 Kpa, and an outlet temperature of the second compressor ranges from 125 to 145°C.

Optionally, in the product rectifying column, a flow rate of the gas phase stream-extracting port of the product rectifying column top is from 1/2 to 3/4 of a flow rate of the feeding port of the product rectifying column.

The present invention adopts the material vapor from the reactive rectifying column top and the hot material as product in the product rectifying column to supply heat for the feeding reaction materials, and thus saves steam consumption; by using the heat pump rectification technology, the vapor from the removal column top is compressed to increase the temperature thereof, and then exchanges heat with the material in the reactive rectifying column to release latent heat, thereby replacing the low pressure steam in the reactive rectifying column reboiler; by using heat pump rectification technology, the vapor from the product rectifying column top is compressed to increase the temperature thereof, and then exchanges heat with the material in the product rectifying column bottom to release latent heat, thereby replacing the low pressure steam in the reactive rectifying column reboiler. And only the reboiler at the removal column bottom consumes low pressure steam, and other column bottoms do not consume low pressure steam. In addition, the product of the present application meets the battery-grade requirements, the quality is stable, no solid hazardous waste containing organic substances is generated, the raw material cost is low, the by-products are few, and the energy is saved.

The beneficial effects that the present application can achieve comprise the followings:
(1) the present invention does not use a disposable homogeneous catalyst, does not need to use a filter to filter out the catalyst, and does not generate solid hazardous waste;
(2) the present invention makes full use of the latent heat of the reactive rectifying column to increase the temperature of the raw material to preheat the raw material, which is one of the energy-saving highlights;
(3) the present invention uses the latent heat of the gas phase stream of the removal column top to provide energy to the reactive rectifying column bottom, thereby saving the steam required by the reactive rectifying column reboiler;
(4) the present invention uses the latent heat of the gas phase stream of the product rectifying column top to provide energy to the product rectifying column bottom, thereby saving the steam required by the product rectifying column reboiler; and
(5) the product of the present invention is extracted from the middle of the product rectifying column, and the product quality is stable.

### BRIEF DESCRIPTION OF DRAWINGS

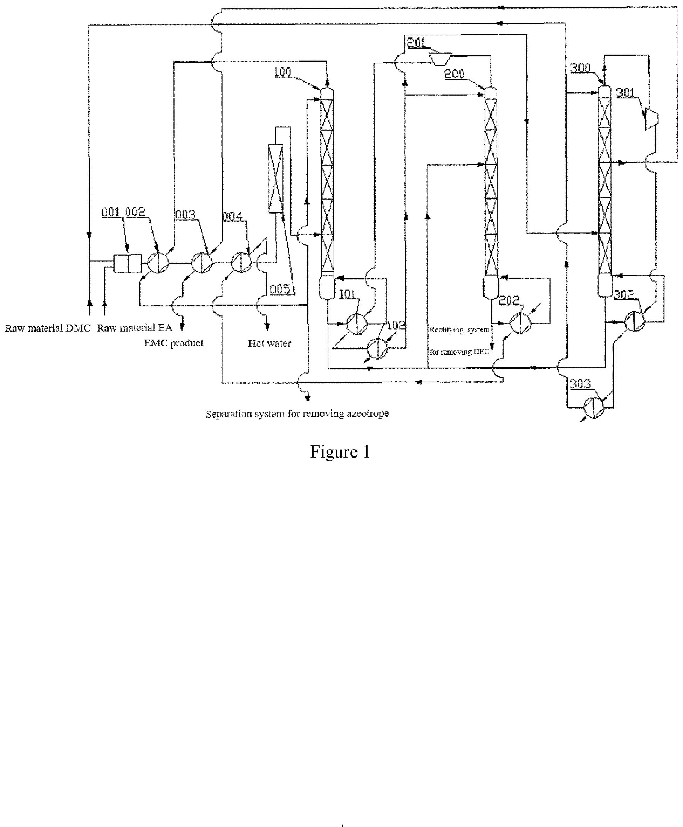

Figure 1 is a schematic structural diagram of the device of the present invention.

The reference signs in Figure 1 are shown as follows: 001 static mixer, 002 first preheater, 003 second preheater, 004 third preheater, 005 pre-reactor, 100 reactive rectifying column, 101 reactive rectifying column reboiler, 200 removal column, 201 first compressor, 202 removal column reboiler, 300 product rectifying column, 301 second compressor, 302 product rectifying column reboiler, 102 first water cooler, 303 second water cooler.

### DETAILED DESCRIPTION

The present application will be described in detail below with reference to the examples, but the present application is not limited to these examples.

Unless otherwise specified, the raw materials in the examples of the present application are all commercially available.

The preferred examples of the present invention are described below.

The present invention provides a device comprising a reactive rectifying column reboiler 101, a first compressor 201, a removal column 200, a product rectifying column 300, a second compressor 301, and a product rectifying column reboiler boiler 302;
the top of the removal column 200 is connected with the reactive rectifying column reboiler 101 through the first compressor 201 so that the gas stream from the top of the removal column 200 is allowed to enter the reactive rectifying column reboiler 101;
the reactive rectifying column reboiler 101 is connected with the removal column 200 and the product rectifying column 300 respectively so that the stream in the reactive rectifying column reboiler 101 is allowed to enter the removal column 200 and the product rectifying column 300;
the top of the product rectifying column 300 is connected with the product rectifying column reboiler 302 through the second compressor 301 so that the gas stream from the top of product rectifying column 300 is allowed to enter the product rectifying column reboiler 302; and
the product rectifying column reboiler 302 is connected with the top of the product rectifying column 300 so that the stream in the product rectifying column reboiler 302 is allowed to enter the product rectifying column 300.

**In** a preferred embodiment, the bottom of the product rectifying column 300 is connected with the product rectifying column reboiler 302 and the removal column 200 respectively, so that the stream in the bottom of the product rectifying column 300 is allowed to enter the product rectifying column reboiler 302 and the removal column 200.

**In** a preferred embodiment, the device further comprises a reactive rectifying column 100, which is connected with the reactive rectifying column reboiler 101 and the removal column 200 respectively so that the stream in the bottom of the reactive rectifying column 100 is allowed to enter the reactive rectifying column reboiler 101 and the removal column 200.

**In** a preferred embodiment, the device further comprises a removal column reboiler 202, and the removal column reboiler 202 is connected with the bottom of the removal column 200 so that the stream in the removal column 200 is allowed to enter the removal column reboiler 202.

**In** a preferred embodiment, the device further comprises a first water cooler 102, the reactive rectifying column reboiler 101 is connected with the removal column 200 through the first water cooler 102, and the reactive rectifying column reboiler 101 is connected with the product rectifying column 300 through the first water cooler 102.

**In** a preferred embodiment, the device further comprises a second water cooler 303, and the product rectifying column reboiler 302 is connected with the top of the product rectifying column 300 through the second water cooler 303.

**In** a preferred embodiment, the device further comprises a pre-reactor 005, and the pre-reactor 005 is connected with the reactive rectifying column 100.

Preferably, the pre-reactor 005 is a tubular fixed bed reactor.

In a preferred embodiment, the device further comprises a preheater and the preheater is connected with the pre-reactor 005.

In a preferred embodiment, the preheater comprises a first preheater 002, a second preheater 003 and a third preheater 004; and the first preheater 002, the second preheater 003 and the third preheater 004 are connected in sequence; the first preheater 002 is connected with the top of the reactive rectifying column 100; the second preheater 003 is connected with the product rectifying column 300; the third preheater 004 is connected with the pre-reactor 005 and the removal column reboiler 202 respectively.

In a preferred embodiment, in the reactive rectifying column 100, the distance between the feeding port and the reflux port is in a range from 2/3 to 4/5 of the total column height; in the removal column 200, the distance between the feeding port and the reflux port is in a range from 1/3 to 1/2 of the height of the total column height; and in the product rectifying column 300, the distance between the feeding port and the reflux port is in a range from 2/3 to 4/5 of the total column height.

In a preferred embodiment, in the product rectifying column 300, the distance between the gas phase stream-extracting port and the reflux port at the top of the product rectifying column 300 is in a range from 1/4 to 1/3 of the total column height.

**In** a preferred embodiment, the device further comprises a static mixer 001 which is connected with the first preheater 002 and the second water cooler 003 respectively, and the static mixer 001 is used for feeding.

**In** a preferred embodiment, the reactive rectifying column 100 is divided into three sections which refer to rectifying section, reaction section and stripping section, the rectifying section comprises from 10 to 30 theoretical plates, the reaction section comprises from 20 to 60 theoretical plates and the stripping section comprises from 10 to 30 theoretical plates.

**In** a preferred embodiment, the number of the theoretical plates of each of the removal column 200 and the product rectifying column 300 is in a range from 30 to 150.

The method for preparing battery-grade ethyl methyl carbonate provided in the present application is carried out by using at least one of the above-mentioned devices.

**In** a preferred embodiment, the method comprises following steps:
(1) passing the material comprising dimethyl carbonate and ethanol into the reactive rectifying column 100 filled with catalyst to perform reaction in the reactive rectifying column 100, and extracting the azeotrope of dimethyl carbonate and ethanol by the gas phase stream-extracting port of the top of the reactive rectifying column 100, and making one part of the mixture I comprising ethyl methyl carbonate, diethyl carbonate, dimethyl carbonate and ethanol enter the reactive rectifying column reboiler 101 and the other part enter the feeding port of the removal column 200;
(2) extracting the mixed steam of ethyl methyl carbonate and dimethyl carbonate from the gas phase stream-extracting port of the top of the removal column 200 which is then subject to compression by the first compressor 201 and is passed into the reactive rectifying column reboiler 101 to supply heat to the reactive rectifying column reboiler 101, wherein a first part of the material entering the reactive rectifying column reboiler 101 is returned to the bottom of the reactive rectifying column 100, and a second part is passed into the reflux port of the top of the removal column 200, and the remaining part is passed into the feeding port of the product rectifying column 300;and
(3) extracting the mixed steam of ethyl methyl carbonate and dimethyl carbonate from the gas phase stream-extracting port of the top of the product rectifying column 300 which is then subject to compression by the second compressor 301 and is passed into the product rectifying column reboiler 302 to supply heat to the product rectifying column reboiler 302, wherein one part of the mixture II comprising diethyl carbonate and ethyl methyl carbonate is passed into the product rectifying column reboiler 302 from the bottom of the product rectifying column 300 and the other part is passed into the feeding port of the removal column 200; a first part of the material entering the product rectifying column reboiler 302 is returned to the bottom of the product rectifying column 300, a second part is returned to the reflux port of the top of the product rectifying column 300, and the remaining part as raw material is passed into the material comprising dimethyl carbonate and ethanol; and the battery-grade ethyl methyl carbonate can be extracted from the middle of the product rectifying column 300.

In a preferred embodiment, the azeotrope of the dimethyl carbonate and ethanol is extracted from the gas phase stream-extracting port of top of the reactive rectifying column 100, then enters into the first preheater 002 where the azeotrope exchanges heat with the material comprising dimethyl carbonate and ethanol, and further then one part of the azeotrope is passed into the reflux port of the top of the reactive rectifying column 100 and the other part is extracted and passed into a separation system for removing azeotrope.

In a preferred embodiment, the method comprises passing the material containing dimethyl carbonate and ethanol into the static mixer 001, then passing the material through the first preheater 002, the second preheater 003 and the third preheater 004 in sequence, and entering the material into the pre-reactor 005 filled with the catalyst to perform the pre-reaction in the pre-reactor 005 followed by entering the resulting material into the feeding port of the reactive rectifying column 100.

In a preferred embodiment, the catalyst is selected from alkaline catalysts.

Preferably, the catalyst comprises a carrier and a metal element, the metal element is supported on the carrier, the metal element is selected from alkali metals; and the carrier is at least one of aluminum oxide, silicon oxide and molecular sieve.

In a preferred embodiment, after the temperature of the material in the reactive rectifying column reboiler 101 is adjusted by the first water cooler 102, one part thereof flows back to the reflux port of the removal column 200, and the other part is passed into the feeding port of the product rectifying column 300.

In a preferred embodiment, after the temperature of the material in the product rectifying column reboiler 302 is adjusted by the second water cooler 303, one part thereof flows back to the reflux port of the product rectifying column 300 and the other part as raw material is passed into the material containing dimethyl carbonate and ethanol.

In a preferred embodiment, the battery-grade ethyl methyl carbonate extracted from the middle of the product rectifying column 300 is passed into the second preheater 003 for cooling and then extracted as the target product.

In a preferred embodiment, one part of the material comprising diethyl carbonate and ethyl methyl carbonate extracted from the bottom of the removal column 200 is passed into the removal column reboiler 202, and the other part is passed into a rectifying system for removing diethyl carbonate; one part of the material entering into the removal column reboiler 202 is returned to the bottom of the removal column 200, and the other part is passed into the third preheater 004; and the removal column reboiler 202 is supplied heat by steam.

In a preferred embodiment, the process operating conditions of the reactive rectifying column 100 are as follows: the temperature of the column top ranges from 60 to 70°C, the pressure of the column bottom ranges from 100 to 150 KPa, the temperature of the column bottom ranges from 80 to 120°C, the pressure of the column bottom ranges from 110 to 200 KPa and the reflux ratio of the column top ranges from 2:1 to 8:1;
the process operating conditions of the removal column 200 are as follows: the temperature of the column top ranges from 80 to 120 °C, the pressure of the column top ranges from 100 to 150 KPa, the temperature of the column bottom ranges from 120 to 140°C, the pressure of the column bottom ranges from 120 to 200 KPa, and the reflux ratio of the column top ranges from 1:1 to 6:1;
the process operating conditions of the product rectifying column 300 are as follows: the temperature of the column top ranges from 90 to 120°C, the pressure of the column top ranges from 100 to 150 KPa, the temperature of the column bottom ranges from 100 to 130°C, the pressure of the column bottom ranges from 120 to 200 KPa, and the reflux ratio of the column top ranges from 2:1 to 8:1; and
the reaction temperature of the pre-reactor 005 ranges from 95 to 105°C.

In a preferred embodiment, in the product rectifying column 300, the flow rate of the gas phase stream-extracting port of the top of the product rectifying column 300 is from 1/2 to 3/4 of the flow rate of the feeding port.

### Example 1

The device in this example comprises a static mixer 001, a first preheater 002, a second preheater 003, a third preheater 004, a pre-reactor 005, a reactive rectifying column 100, a reactive rectifying column reboiler 101, a removal column 200, a first compressor 201, a removal column reboiler 202, a product rectifying column 300, a second compressor 301, a product rectifying column reboiler 302, a first water cooler 102 and a second water cooler 303, wherein the inlet of the static mixer 001 is connected with the raw material DMC feeding pipeline and the raw material EA feeding pipeline respectively, the outlet of the static mixer 001 is connected with the tube side inlet of the first preheater 002, the tube side outlet of the first preheater 002 is connected with the tube side inlet of the second preheater 003, the outlet of the second preheater 003 is connected with the outlet of the third preheater 004, the outlet of the third preheater 004 is connected with the lower port of the pre-reactor 005, and the upper outlet of the pre-reactor 005 is connected with the feeding port of the reactive rectifying column 100;
the upper gas phase stream outlet of the reactive rectifying column 100 is connected with the shell side inlet of the first preheater 002, and the shell side outlet of the first preheater 002 is divided into two paths wherein one is connected with the upper reflux port of the reactive rectifying column 100 and the other is connected with the production pipeline; the lower column bottom outlet of the reactive rectifying column 100 is connected with the production pipeline of column bottom and the tube side inlet of the reactive rectifying column reboiler 101 respectively, and the tube side outlet of the reactive rectifying column reboiler 101 is connected with the column bottom; the production pipeline of the column bottom is connected with the middle of the removal column 200;
the lower port of the bottom of the removal column 200 is connected with the DEC production line and the tube side inlet of the removal column reboiler 202 respectively, and the tube side outlet of the removal column reboiler 202 is connected with the column bottom, the gas phase stream port of the top of the removal column 200 is connected with the inlet of the first compressor 201, the outlet of the first compressor 201 is connected with the shell side inlet of the reactive rectifying column reboiler 101, the shell side outlet of the reactive rectifying column reboiler 101 is connected with the inlet of the first water cooler 102, and the material after being subject to cooling down by the first water cooler 102 enters the middle of the product rectifying column 300; and
the gas phase stream port of the top of the product rectifying column 300 is connected with the inlet of the second compressor 301, the outlet of the second compressor 301 is connected with the inlet of the product rectifying column reboiler 302, the outlet of the product rectifying column reboiler 302 is connected with the second water cooler 303, and the outlet pipeline of the second water cooler 303 is divided into two paths wherein one is connected with the reflux port of the product rectifying column 300 and the other is connected with the feeding line of dimethyl carbonate; the inlet of the product rectifying column reboiler 302 is connected with the lower port of the bottom of the product rectifying column 300, and the outlet of the product rectifying column reboiler 302 is connected with the column bottom.

The reaction temperature of the mixed raw materials in the pre-reactor 005 after passing through the first pre-heater, the second pre-heater and the third pre-heater ranges from 60 to 120°C; the pre-reactor 005 is tubular fixed bed reactor and is filled with solid catalyst which is a medium-alkaline catalyst, and the shape thereof is spherical; the feeding port is opened in a tray distributor between the reaction section and the stripping section, and the reaction is carried out in the high concentration area of dimethyl carbonate and anhydrous ethanol above the feeding port.

The method for preparing the catalyst used in this Example is as follows: weighing by weight 100 parts of γ-Al₂O₃ powder, slowly adding therein 36 parts of potassium fluoride solution with 20% mass percentage concentration, 33 parts of cesium nitrate solution with 16% mass percentage concentration, and 32 parts of potassium nitrate solution with 15% mass percentage concentration, stirring uniformly, extruding and cutting the resulting sample into strips with a diameter of 2.5 mm and a length of 8 mm; putting the strips in an oven at 80°C to perform drying for 10 hours, and heating the oven to 150°C to perform further drying for 5 hours, then putting the dried sample into a 300°C muffle furnace to perform activation for 8 hours, heating the muffle furnace to 760°C to perform further activation for 10 hours, cooling down the activated sample, and putting the same into a dry environment to perform natural cooling for later use.

The method for preparing battery-grade ethyl methyl carbonate by using the device in this Example comprises following steps:
A, passing dimethyl carbonate and anhydrous alcohol into the static mixer 001 according to a molar ratio of 1.4:1 and then into the multi-stage preheater to be preheated to the reaction temperature of 96°C, and then into the pre-reactor 005 to perform pre-reaction;
B. passing the resulting material from the pre-reactor 005 into the reactive rectifying column 100 to perform the catalytic rectifying reaction and evaporating the azeotrope of dimethyl carbonate and methanol from the top of the reactive rectifying column 100, wherein after the azeotrope exchanges heat with the feeding material, one part of the azeotrope flows back into the reactive rectifying column and the other part is extracted to the outside the reactive rectifying column; and the mixed ester is extracted from the bottom of the reaction rectifying column 100 to the removal column 200; and the temperature of the top of the reactive rectifying column 100 is 65.3 °C, the pressure of the top pf the reactive rectifying column 100 is 101 KPa, the temperature of the bottom of the reactive rectifying column 100 is 98 °C, the pressure of the bottom of the reactive rectifying column 100 is 113 KPa, and the reflux ratio of the top of the reactive rectifying column 100 is 4.1:1;
C, supplying heat to the removal column reboiler 202 with low pressure steam and supplying heat to the reactive rectifying column reboiler 101 with the steam from removal column top after it is heated and pressurized by the first compressor 201, wherein, after the temperature of the steam is adjusted by the first water cooler 102, one part thereof flows back to the removal column 200 and the other part is extracted into the product rectifying column 300; and the temperature of the top of the removal column 200 is 95 °C, the pressure of the top of the removal column 200 is 102 KPa, the temperature of the bottom of the removal column 200 is 129 °C, the pressure of the bottom of the removal column 200 is 108 KPa, and the distance between the feeding port and the reflux port is 0.42 of the total column height; the number of theoretical plates of the rectifying column is 65 and the reflux ratio of the column top is 1.1; and the compression ratio of the first compressor is 2.5, the outlet pressure is 253 KPa, and the outlet temperature is 127°C;
D, supplying heat to the product rectifying column reboiler 302 with the component from the top of the product rectifying column 300 after it is heated and pressurized by the second compressor 301, wherein, after the temperature of the component is adjusted by the second water cooler 303, one part flows back to the product rectifying column and the other part as feeding material is extracted to the reaction system; and the battery-grade ethyl methyl carbonate extracted from the product rectifying column is preheated by the preheater and then extracted as product; and
E, the temperature of the top of the product rectifying column 300 is 103°C, and the pressure of the top of the product rectifying column 300 is 102 KPa; the temperature of the bottom of the product rectifying column is 110°C, the pressure of the bottom of the product rectifying column is 108 KPa, the distance between the feeding port and the reflux port is 0.71 of the total column height, the distance between the extracting port and the reflux port is 0.28 of the total column height, the extracting flow rate is 0.63 of the feeding flow rate, the number of theoretical plates of the rectifying column is 96, the reflux ratio is 4.8:1, the compression ratio of the second compressor 301 at the top of the product rectifying column 300 is 2.9, the outlet pressure of the second compressor 301 is 303 KPa, and the outlet temperature of the second compressor 301 is 135 °C.

The ethyl methyl carbonate as product obtained in this example is sampled with a closed sampler, and the moisture therein is measured with a WS-2100 trace moisture analyzer, and the average value of three measurements is 0.0012%. Shimadzu GC2010plus gas chromatograph and its own data processing workstation, DB-WAX quartz glass capillary column, programmed temperature, area normalization method as data processing method are used to perform test and the test results are as follows: methanol content is 0.0018%, ethanol content is 0.0016% and ethyl methyl carbonate content is 99.992%, which exceed the battery-grade quality requirements.

In a dry glove box, 20 g lithium hexafluorophosphate is weighed and dissolved in a preweighed 80 g the above product in a 200 mL PTFE bottle with a lid, then stirring step is operated with PTFE stirring rod to dissolve and stir evenly, and the resulting solution is put in an oven to bake at pre-stabilized 60 °C for 8 hours. After taking the baked sample out from the oven, it is put into a closed glass desiccator to cool for 2 hours, and then put into an OME2000 colorimeter to test the chromaticity. The chromaticity result is 10, which is better than the control index.

### Example 2

The preparation device is identical to that in Example 1, and the preparation process is identical to that in Example 1, and the specific operating conditions are as follows:
passing dimethyl carbonate and anhydrous alcohol into the static mixer 001 according to a molar ratio of 1.2:1 and then into the first preheater 002, the second preheater 003 and the third preheater 004 to be preheated to the reaction temperature of 102°C, and then into the pre-reactor 005 to perform pre-reaction;
the temperature of the top of the reactive rectifying column 100 is 64.6 °C, the pressure of the top pf the reactive rectifying column 100 is 103 KPa, the temperature of the bottom of the reactive rectifying column 100 is 103 °C, the pressure of the bottom of the reactive rectifying column 100 is 108 KPa, and the reflux ratio of the top of the reactive rectifying column 100 is 4.3:1;
the temperature of the top of the removal column 200 is 98 °C, the pressure of the top of the removal column 200 is 102 KPa, the temperature of the bottom of the removal column 200 is 131 °C, the pressure of the bottom of the removal column 200 is 106 KPa, and the distance between the feeding port and the reflux port is 0.42 of the total column height; the number of theoretical plates of the rectifying column is 72 and the reflux ratio of the column top is 1.30; and the compression ratio of the compressor at the top of the removal column 200 is 2.8, the outlet pressure is 258 KPa, and the outlet temperature is 128°C; and
the temperature of the top of the product rectifying column 300 is 106°C, and the pressure of the top of the product rectifying column 300 is 100 KPa; the temperature of the bottom of the product rectifying column is 110°C, the pressure of the bottom of the product rectifying column is 105 KPa, the distance between the feeding port and the reflux port is 0.71 of the total column height, the distance between the extracting port and the reflux port is 0.28 of the total column height, the extracting flow rate is 0.60 of the feeding flow rate, the number of theoretical plates of the rectifying column is 99, the reflux ratio is 5.2:1, and the compression ratio of the second compressor 301 at the top of the product rectifying column 300 is 3.2, the outlet pressure of the second compressor 301 is 323 KPa, and the outlet temperature of the second compressor 301 is 140 °C.

The ethyl methyl carbonate as product obtained in this example is sampled with a closed sampler, and the moisture therein is measured with a WS-2100 trace moisture analyzer, and the average value of three measurements is 0.0008%. Shimadzu GC2010plus gas chromatograph and its own data processing workstation, DB-WAX quartz glass capillary column, programmed temperature, area normalization method as data processing method are used to perform test and the test results are as follows: methanol content is 0.0008%, ethanol content is 0.0006% and ethyl methyl carbonate content is 99.993%, which exceed the battery-grade quality requirements.

According to the method and identical to those in Example 1, the product obtained in Example 2 is tested, and the chromaticity result is 11, which is better than the control index.

Table 1 is a comparison table of product quality and energy consumption before implementation, Example 1 and Example 2.

**Table 1**

| Number | Ethyl methyl carbonate | Power consumpti on per ton of product | Steam consumptio n per ton of product | Energy consumption per ton of product | Energy saving percenta ge | Energy cost | Catalyst cost | Total cost |
|---|---|---|---|---|---|---|---|---|
| | Quality grade | (KWh/t) | (t/t) | (converted to standard coal) | (%) | (yuan/t) | (yuan/t) | (yuan/t) |
| Before implementation | battery-grade | 100 | 5.2 | 0.480 | ---- | 865 | 60 | 925 |
| Example 1 | battery-grade | 160 | 3.0 | 0.290 | 39.6 | 586 | 150 | 736 |
| Example 2 | battery-grade | 168 | 2.9 | 0.282 | 41.3 | 578 | 150 | 728 |

In the above table, the power electricity is calculated at 0.85 yuan/kWh, and the low pressure steam is calculated at 150 yuan/t.

It can be seen from the above table that the average processing cost after the implementation is 732 yuan/t, which is 193 yuan/t lower than that before the implementation. The annual cost for the 30,000 tons/year preparation device is reduced by 5.79 million yuan, and the economic benefits are obvious.

## Claims

1. A method for preparing battery-grade ethyl methyl carbonate which is carried out by using at least one device of:
device I comprising:
a reactive rectifying column reboiler (101), a first compressor (201), a removal column (200), a product rectifying column (300), a second compressor (301) and a product rectifying column reboiler (302);
wherein
a top of the removal column is connected with the reactive rectifying column reboiler through the first compressor so that a gas stream from the top of the removal column is allowed to enter the reactive rectifying column reboiler;
the reactive rectifying column reboiler is connected with the removal column and the product rectifying column respectively so that the gas stream in the reactive rectifying column reboiler is allowed to enter the removal column and the product rectifying column;
a top of the product rectifying column is connected with the product rectifying column reboiler through the second compressor so that the gas stream from the top of the product rectifying column is allowed to enter the product rectifying column reboiler; and
the product rectifying column reboiler is connected with the top of the product rectifying column so that the gas stream in the product rectifying column reboiler is allowed to enter the product rectifying column;
wherein the device further comprises a first water cooler (102), the reactive rectifying column reboiler is connected with the removal column through the first water cooler; and the reactive rectifying column reboiler is connected with the product rectifying column through the first water cooler;
and
device II comprising:
a reactive rectifying column reboiler (101), a first compressor (201), a removal column (200), a product rectifying column (300), a second compressor (301) and a product rectifying column reboiler (302);
wherein
a top of the removal column is connected with the reactive rectifying column reboiler through the first compressor so that a gas stream from the top of the removal column is allowed to enter the reactive rectifying column reboiler;
the reactive rectifying column reboiler is connected with the removal column and the product rectifying column respectively so that the gas stream in the reactive rectifying column reboiler is allowed to enter the removal column and the product rectifying column;
a top of the product rectifying column is connected with the product rectifying column reboiler through the second compressor so that the gas stream from the top of the product rectifying column is allowed to enter the product rectifying column reboiler; and
the product rectifying column reboiler is connected with the top of the product rectifying column so that the gas stream in the product rectifying column reboiler is allowed to enter the product rectifying column;
wherein the device further comprises a second water cooler (303), and the product rectifying column reboiler is connected with the top of the product rectifying column through the second water cooler;
wherein the method comprises the following steps:
(1) passing a material comprising dimethyl carbonate and ethanol into a reactive rectifying column filled with a catalyst to perform a reaction in the reactive rectifying column, and extracting azeotrope of dimethyl carbonate and ethanol by a gas phase stream-extracting port of a top of the reactive rectifying column, and making one part of a mixture I comprising ethyl methyl carbonate, diethyl carbonate, dimethyl carbonate and ethanol enter the reactive rectifying column reboiler and the other part of the mixture I enter a feeding port of the removal column;
(2) extracting a mixed steam of ethyl methyl carbonate and dimethyl carbonate from a gas phase stream-extracting port of the top of the removal column which is then subject to compression by the first compressor and is passed into the reactive rectifying column reboiler to supply heat to the reactive rectifying column reboiler, wherein a first part of the material entering the reactive rectifying column reboiler is returned to a bottom of the reactive rectifying column, a second part thereof is passed into a reflux port of the top of the removal column, and the remaining part thereof is passed into a feeding port of the product rectifying column;
(3) extracting a mixed steam of ethyl methyl carbonate and dimethyl carbonate from a gas phase stream-extracting port of the top of the product rectifying column which is then subject to compression by the second compressor and is passed into the product rectifying column reboiler to supply heat to the product rectifying column reboiler, wherein one part of a mixture II comprising diethyl carbonate and ethyl methyl carbonate is passed into the product rectifying column reboiler from a bottom of the product rectifying column and the other part is passed into the feeding port of the removal column; a first part of the material entering the product rectifying column reboiler is returned to the bottom of the product rectifying column, a second part thereof is returned to a reflux port of the top of the product rectifying column, and the remaining part thereof as raw material is passed into the material comprising dimethyl carbonate and ethanol; and the battery-grade ethyl methyl carbonate can be extracted from a middle of the product rectifying column;
the azeotrope of dimethyl carbonate and ethanol is extracted from the gas phase stream-extracting port of the top of the reactive rectifying column, then enters into a first preheater where the azeotrope exchanges heat with the material comprising dimethyl carbonate and ethanol, and further then one part of the azeotrope is passed into a reflux port of the top of the reactive rectifying column and the other part is extracted and passed into a separation system for removing the azeotrope;
the method comprises passing the material containing dimethyl carbonate and ethanol into a static mixer, then passing the material through the first preheater, a second preheater and a third preheater in sequence, and entering the material into a pre-reactor filled with the catalyst to perform a pre-reaction in the pre-reactor followed by entering a resulting material into a feeding port of the reactive rectifying column.

2. The method according to claim 1, wherein the catalyst is selected from alkaline catalysts;
the catalyst comprises a carrier and a metal element, and the metal element is supported on the carrier; the metal element is selected from alkali metals; and the carrier is at least one of aluminum oxide, silicon oxide and molecular sieve.

3. The method according to claim 1, wherein
when the device I is used, after the temperature of the material in the reactive rectifying column reboiler is adjusted by the first water cooler, one part thereof flows back to the reflux port of the removal column, and the other part thereof is passed into the feeding port of the product rectifying column;
when the device II is used, after the temperature of the material in the product rectifying column reboiler is adjusted by the second water cooler, one part thereof flows back to the reflux port of the product rectifying column and the other part as raw material is passed into the material containing dimethyl carbonate and ethanol.

4. The method according to claim 1, wherein the battery-grade ethyl methyl carbonate extracted from the middle of the product rectifying column is passed into the second preheater for cooling and then extracted as a target product.

5. The method according to claim 1, wherein one part of the material comprising diethyl carbonate and ethyl methyl carbonate extracted from a bottom of the removal column is passed into a removal column reboiler, and the other part is passed into a rectifying system for removing diethyl carbonate,
one part of the material entering the removal column reboiler is returned to the bottom of the removal column, and the other part is passed into the third preheater; and
the removal column reboiler is supplied heat by steam.

6. The method according to claim 1, wherein process operating conditions of the reactive rectifying column are as follows: a temperature of the top of the reactive rectifying column ranges from 60 to 70°C, a pressure of the bottom of the reactive rectifying column ranges from 100 to 150 KPa, a temperature of the bottom of the reactive rectifying column ranges from 80 to 120 °C, a pressure of the bottom of the reactive rectifying column ranges from 110 to 200 KPa and a reflux ratio of the top of the reactive rectifying column ranges from 2:1 to 8:1;
process operating conditions of the removal column are as follows: a temperature of the top of the removal column ranges from 80 to 120 °C, a pressure of the top of the removal column ranges from 100 to 150 KPa, a temperature of a bottom of the removal column ranges from 120 to 140 °C, a pressure of the bottom of the removal column ranges from 120 to 200 KPa, and a reflux ratio of the top of the removal column ranges from 1:1 to 6:1;
process operating conditions of the product rectifying column are as follows: a temperature of the top of the product rectifying column ranges from 90 to 120 °C, a pressure of the top of the product rectifying column ranges from 100 to 150 KPa, a temperature of the bottom of the product rectifying column ranges from 100 to 130°C, a pressure of the bottom of the product rectifying column ranges from 120 to 200 KPa, and a reflux ratio of the top of the product rectifying column ranges from 2:1 to 8:1; and
a reaction temperature of the pre-reactor ranges from 90 to 120°C.

7. The method according to claim 1, wherein in the material comprising dimethyl carbonate and ethanol, a molar ratio of the dimethyl carbonate to the ethanol ranges from 0.5:1 to 2:1;
a compression ratio of the first compressor ranges from 2 to 3, an outlet pressure of the first compressor ranges from 200 to 300 KPa, and an outlet temperature of the first compressor ranges from 120 to 145°C; and a compression ratio of the second compressor ranges from 2.0 to 3.5; an outlet pressure of the second compressor ranges from 200 to 360 Kpa, and an outlet temperature of the second compressor ranges from 125 to 145°C;
in the product rectifying column, a flow rate of the gas phase stream-extracting port of the top of the product rectifying column is from 1/2 to 3/4 of a flow rate of the feeding port of the product rectifying column.

## Patentansprüche

1. Verfahren zur Herstellung von Ethylmethylcarbonat in Batteriequalität, das unter Nutzung von mindestens einer der folgenden Vorrichtungen durchgeführt wird:
Vorrichtung I, umfassend:
einen reaktiven Rektifikationskolonnen-Verdampfer (101), einen ersten Kompressor (201), eine Trennkolonne (200), eine Produktrektifikationskolonne (300), einen zweiten Kompressor (301) und einen Produktrektifikationskolonnen-Verdampfer (302),
wobei
eine Oberseite der Trennkolonne durch den ersten Kompressor mit dem reaktiven Rektifikationskolonnen-Verdampfer verbunden ist, sodass ein Gasstrom von der Oberseite der Trennkolonne in den reaktiven Rektifikationskolonnen-Verdampfer einströmen kann;
der reaktive Rektifikationskolonnen-Verdampfer jeweils mit der Trennkolonne und der Produktrektifikationskolonne verbunden ist, sodass der Gasstrom im reaktiven Rektifikationskolonnen-Verdampfer in die Trennkolonne und die Produktrektifikationskolonne einströmen kann;
eine Oberseite der Produktrektifikationskolonne durch den zweiten Kompressor mit dem Produktrektifikationskolonnen-Verdampfer verbunden ist, sodass der Gasstrom von der Oberseite der Produktrektifikationskolonne in den Produktrektifikationskolonnen-Verdampfer einströmen kann, und
der Produktrektifikationskolonnen-Verdampfer mit der Oberseite der Produktrektifikationskolonne verbunden ist, sodass der Gasstrom im Produktrektifikationskolonnen-Verdampfer in die Produktrektifikationskolonne einströmen kann,
wobei die Vorrichtung zudem einen ersten Wasserkühler (102) umfasst, der reaktive Rektifikationskolonnen-Verdampfer durch den ersten Wasserkühler mit der Trennkolonne verbunden ist und der reaktive Rektifikationskolonnen-Verdampfer durch den ersten Wasserkühler mit der Produktrektifikationskolonne verbunden ist,
und
Vorrichtung II, umfassend:
einen reaktiven Rektifikationskolonnen-Verdampfer (101), einen ersten Kompressor (201), eine Trennkolonne (200), eine Produktrektifikationskolonne (300), einen zweiten Kompressor (301) und einen Produktrektifikationskolonnen-Verdampfer (302),
wobei
eine Oberseite der Trennkolonne durch den ersten Kompressor mit dem reaktiven Rektifikationskolonnen-Verdampfer verbunden ist, sodass ein Gasstrom von der Oberseite der Trennkolonne in den reaktiven Rektifikationskolonnen-Verdampfer einströmen kann;
der reaktive Rektifikationskolonnen-Verdampfer jeweils mit der Trennkolonne und der Produktrektifikationskolonne verbunden ist, sodass der Gasstrom im reaktiven Rektifikationskolonnen-Verdampfer in die Trennkolonne und die Produktrektifikationskolonne einströmen kann;
eine Oberseite der Produktrektifikationskolonne durch den zweiten Kompressor mit dem Produktrektifikationskolonnen-Verdampfer verbunden ist, sodass der Gasstrom von der Oberseite der Produktrektifikationskolonne in den Produktrektifikationskolonnen-Verdampfer einströmen kann, und
der Produktrektifikationskolonnen-Verdampfer mit der Oberseite der Produktrektifikationskolonne verbunden ist, sodass der Gasstrom im Produktrektifikationskolonnen-Verdampfer in die Produktrektifikationskolonne einströmen kann,
wobei die Vorrichtung zudem einen zweiten Wasserkühler (303) umfasst und der Produktrektifikationskolonnen-Verdampfer durch den zweiten Wasserkühler mit der Oberseite der Produktrektifikationskolonne verbunden ist,
wobei das Verfahren die folgenden Schritte umfasst:
(1) Leiten eines Materials, umfassend Dimethylcarbonat und Ethanol, in eine mit einem Katalysator gefüllte reaktive Rektifikationskolonne, um eine Reaktion in der reaktiven Rektifikationskolonne durchzuführen, und Extrahieren von Azeotrop aus Dimethylcarbonat und Ethanol durch eine Gasphasenstrom-Extraktionsöffnung einer Oberseite der reaktiven Rektifikationskolonne, und Bewirken, dass ein Teils eines Gemischs I, umfassend Ethylmethylcarbonat, Diethylcarbonat, Dimethylcarbonat und Ethanol, in den reaktiven Rektifikationskolonnen-Verdampfer einströmt und der andere Teil des Gemischs I in eine Zufuhröffnung der Trennkolonne einströmt;
(2) Extrahieren eines gemischten Dampfs aus Ethylmethylcarbonat und Dimethylcarbonat aus einer Gasphasenstrom-Extraktionsöffnung der Oberseite der Trennkolonne, der dann der Kompression durch den ersten Kompressor unterzogen und in den reaktiven Rektifikationskolonnen-Verdampfer geleitet wird, um dem reaktiven Rektifikationskolonnen-Verdampfer Wärme zuzuführen, wobei ein erster Teil des Materials, das in den reaktiven Rektifikationskolonnen-Verdampfer strömt, zu einer Unterseite der reaktiven Rektifikationskolonne zurückgeführt wird, ein zweiter Teil davon in eine Rückflussöffnung der Oberseite der Trennkolonne geleitet wird und der verbleibende Teil davon in eine Zufuhröffnung der Produktrektifikationskolonne geleitet wird;
(3) Extrahieren eines gemischten Dampfs aus Ethylmethylcarbonat und Dimethylcarbonat aus einer Gasphasenstrom-Extraktionsöffnung der Oberseite der Produktrektifikationskolonne, der dann der Kompression durch den zweiten Kompressor unterzogen und in den Produktrektifikationskolonnen-Verdampfer geleitet wird, um dem Produktrektifikationskolonnen-Verdampfer Wärme zuzuführen, wobei ein Teil eines Gemischs II, umfassend Diethylcarbonat und Ethylmethylcarbonat, von einer Unterseite der Produktrektifikationskolonne in den Produktrektifikationskolonnen-Verdampfer geleitet wird und der andere Teil in die Zufuhröffnung der Trennkolonne geleitet wird, ein erster Teil des Materials, das in den Produktrektifikationskolonnen-Verdampfer einströmt, zur Unterseite der Produktrektifikationskolonne zurückgeführt wird, ein zweiter Teil davon zu einer Rückflussöffnung der Oberseite der Produktrektifikationskolonne zurückgeführt wird und der verbleibende Teil davon als Rohmaterial in das Material, umfassend Dimethylcarbonat und Ethanol, geleitet wird und das Ethylmethylcarbonat in Batteriequalität aus einer Mitte der Produktrektifikationskolonne extrahiert werden kann,
wobei das Azeotrop aus Dimethylcarbonat und Ethanol aus der Gasphasenstrom-Extraktionsöffnung der Oberseite der reaktiven Rektifikationskolonne extrahiert wird, dann in einen ersten Vorwärmer einströmt, in dem das Azeotrop Wärme mit dem Material, umfassend Dimethylcarbonat und Ethanol, austauscht, und dann ein Teil des Azeotrops in eine Rückflussöffnung der Oberseite der reaktiven Rektifikationskolonne geleitet und der andere Teil extrahiert und in ein Trennsystem zum Trennen des Azeotrops geleitet wird,
wobei das Verfahren das Leiten des Dimethylcarbonat und Ethanol enthaltenden Materials in einen statischen Mischer, dann das Leiten des Materials nacheinander durch den ersten Vorwärmer, einen zweiten Vorwärmer und einen dritten Vorwärmer und das Einführen des Materials in einen mit dem Katalysator gefüllten Vorreaktor umfasst, um eine Vorreaktion im Vorreaktor durchzuführen, gefolgt vom Einführen eines hervorgegangenen Materials in eine Zufuhröffnung der reaktiven Rektifikationskolonne.

2. Verfahren nach Anspruch 1, wobei der Katalysator aus alkalischen Katalysatoren ausgewählt ist,
der Katalysator einen Träger und ein Metallelement umfasst und das Metallelement auf dem Träger getragen wird, das Metallelement aus Alkalimetallen ausgewählt ist und der Träger mindestens entweder Aluminiumoxid und/oder Siliziumoxid und/oder Molekularsieb ist.

3. Verfahren nach Anspruch 1, wobei
bei der Verwendung von Vorrichtung I nach der Regelung der Temperatur des Materials im reaktiven Rektifikationskolonnen-Verdampfer durch den ersten Wasserkühler ein Teil davon zurück zur Rückflussöffnung der Trennkolonne strömt und der andere Teil davon in die Zufuhröffnung der Produktrektifikationskolonne geleitet wird;
bei der Verwendung von Vorrichtung II nach der Regelung der Temperatur des Materials im Produktrektifikationskolonnen-Verdampfer durch den zweiten Wasserkühler ein Teil davon zurück zur Rückflussöffnung der Produktrektifikationskolonne strömt und der andere Teil davon als Rohmaterial in das Dimethylcarbonat und Ethanol enthaltende Material geleitet wird.

4. Verfahren nach Anspruch 1, wobei das aus der Mitte der Produktrektifikationskolonne extrahierte Ethylmethylcarbonat in Batteriequalität zum Kühlen in den zweiten Vorwärmer geleitet und dann als Zielprodukt extrahiert wird.

5. Verfahren nach Anspruch 1, wobei ein Teil des von einer Unterseite der Trennkolonne extrahierten Materials, umfassend Diethylcarbonat und Ethylmethylcarbonat, in einen Trennkolonnen-Verdampfer geleitet wird und der andere Teil in ein Rektifikationssystem zum Trennen von Diethylcarbonat geleitet wird,
ein Teil des in den Trennkolonnen-Verdampfer einströmenden Materials zur Unterseite der Trennkolonne zurückgeführt wird und der andere Teil in den dritten Vorwärmer geleitet wird und
dem Trennkolonnen-Verdampfer Wärme durch Dampf zugeführt wird.

6. Verfahren nach Anspruch 1, wobei die Prozessbetriebsbedingungen der reaktiven Rektifikationskolonne wie folgt sind: Eine Temperatur der Oberseite der reaktiven Rektifikationskolonne liegt im Bereich von 60 bis 70 °C, ein Druck der Unterseite der reaktiven Rektifikationskolonne liegt im Bereich von 100 bis 150 KPa, eine Temperatur der Unterseite der reaktiven Rektifikationskolonne liegt im Bereich von 80 bis 120 °C, ein Druck der Unterseite der reaktiven Rektifikationskolonne liegt im Bereich von 110 bis 200 KPa und ein Rückflussverhältnis der Oberseite der reaktiven Rektifikationskolonne liegt im Bereich von 2:1 bis 8:1,
wobei die Prozessbetriebsbedingungen der Trennkolonne wie folgt sind: Eine Temperatur der Oberseite der Trennkolonne liegt im Bereich von 80 bis 120 °C, ein Druck der Oberseite der Trennkolonne liegt im Bereich von 100 bis 150 KPa, eine Temperatur einer Unterseite der Trennkolonne liegt im Bereich von 120 bis 140 °C, ein Druck der Unterseite der Trennkolonne liegt im Bereich von 120 bis 200 KPa und ein Rückflussverhältnis der Oberseite der Trennkolonne liegt im Bereich von 1:1 bis 6:1,
wobei die Prozessbetriebsbedingungen der Produktrektifikationskolonne wie folgt sind: Eine Temperatur der Oberseite der Produktrektifikationskolonne liegt im Bereich von 90 bis 120 °C, ein Druck der Oberseite der Produktrektifikationskolonne liegt im Bereich von 100 bis 150 KPa, eine Temperatur der Unterseite der Produktrektifikationskolonne liegt im Bereich von 100 bis 130 °C, ein Druck der Unterseite der Produktrektifikationskolonne liegt im Bereich von 120 bis 200 KPa und ein Rückflussverhältnis der Oberseite der Produktrektifikationskolonne liegt im Bereich von 2:1 bis 8:1 und
eine Reaktionstemperatur des Vorreaktors liegt im Bereich von 90 bis 120 °C.

7. Verfahren nach Anspruch 1, wobei in dem Material, umfassend Dimethylcarbonat und Ethanol, ein Molverhältnis von Dimethylcarbonat zu Ethanol im Bereich von 0,5:1 bis 2:1 liegt,
ein Verdichtungsverhältnis des ersten Kompressors im Bereich von 2 bis 3 liegt, ein Ausgangsdruck des ersten Kompressors im Bereich von 200 bis 300 KPa liegt und eine Austrittstemperatur des ersten Kompressors im Bereich von 120 bis 145 °C liegt und ein Verdichtungsverhältnis des zweiten Kompressors im Bereich von 2,0 bis 3,5 liegt, ein Ausgangsdruck des zweiten Kompressors im Bereich von 200 bis 360 KPa liegt und eine Austrittstemperatur des zweiten Kompressors im Bereich von 125 bis 145 °C liegt,
in der Produktrektifikationskolonne eine Durchflussrate der Gasphasenstrom-Extraktionsöffnung der Oberseite der Produktrektifikationskolonne 1/2 bis 3/4 einer Durchflussrate der Zufuhröffnung der Produktrektifikationskolonne beträgt.

## Revendications

1. Procédé de préparation de carbonate de méthyle d'éthyle de qualité pour batterie qui est réalisé en utilisant au moins un dispositif parmi :
un dispositif I comprenant :
un rebouilleur de colonne de rectification de réactif (101), un premier compresseur (201), une colonne d'élimination (200), une colonne de rectification de produit (300), un deuxième compresseur (301) et un rebouilleur de colonne de rectification de produit (302) ;
dans lequel
une partie supérieure de la colonne d'élimination est reliée au rebouilleur de colonne de rectification de réactif à travers le premier compresseur de sorte qu'un flux de gaz provenant de la partie supérieure de la colonne d'élimination est autorisé à entrer dans le rebouilleur de colonne de rectification de réactif ;
le rebouilleur de colonne de rectification de réactif est relié à la colonne d'élimination et à la colonne de rectification de produit respectivement de sorte que le flux de gaz dans le rebouilleur de colonne de rectification de réactif est autorisé à entrer dans la colonne d'élimination et la colonne de rectification de produit ;
une partie supérieure de la colonne de rectification de produit est reliée au rebouilleur de colonne de rectification de produit à travers le deuxième compresseur de sorte que le flux de gaz provenant de la partie supérieure de la colonne de rectification de produit est autorisé à entrer dans le rebouilleur de colonne de rectification de produit ; et
le rebouilleur de colonne de rectification de produit est relié à la partie supérieure de la colonne de rectification de produit de sorte que le flux de gaz dans le rebouilleur de colonne de rectification de produit est autorisé à entrer dans la colonne de rectification de produit ;
dans lequel le dispositif comprend en outre un premier refroidisseur d'eau (102), le rebouilleur de colonne de rectification de réactif est relié à la colonne d'élimination à travers le premier refroidisseur d'eau ; et le rebouilleur de colonne de rectification de réactif est relié à la colonne de rectification de produit à travers le premier refroidisseur d'eau ;
et
un dispositif II comprenant :
un rebouilleur de colonne de rectification de réactif (101), un premier compresseur (201), une colonne d'élimination (200), une colonne de rectification de produit (300), un deuxième compresseur (301) et un rebouilleur de colonne de rectification de produit (302) ;
dans lequel
une partie supérieure de la colonne d'élimination est reliée au rebouilleur de colonne de rectification de réactif à travers le premier compresseur de sorte qu'un flux de gaz provenant de la partie supérieure de la colonne d'élimination est autorisé à entrer dans le rebouilleur de colonne de rectification de réactif ;
le rebouilleur de colonne de rectification de réactif est relié à la colonne d'élimination et à la colonne de rectification de produit respectivement de sorte que le flux de gaz dans le rebouilleur de colonne de rectification de réactif est autorisé à entrer dans la colonne d'élimination et la colonne de rectification de produit ;
une partie supérieure de la colonne de rectification de produit est reliée au rebouilleur de colonne de rectification de produit à travers le deuxième compresseur de sorte que le flux de gaz provenant de la partie supérieure de la colonne de rectification de produit est autorisé à entrer dans le rebouilleur de colonne de rectification de produit ; et
le rebouilleur de colonne de rectification de produit est relié à la partie supérieure de la colonne de rectification de produit de sorte que le flux de gaz dans le rebouilleur de colonne de rectification de produit est autorisé à entrer dans la colonne de rectification de produit ;
dans lequel le dispositif comprend en outre un deuxième refroidisseur d'eau (303), et le rebouilleur de colonne de rectification de produit est relié à la partie supérieure de la colonne de rectification de produit à travers le deuxième refroidisseur d'eau ;
dans lequel le procédé comprend les étapes suivantes :
(1) le passage d'une matière comprenant du carbonate de diméthyle et de l'éthanol dans une colonne de rectification de réactif remplie de catalyseur pour effectuer une réaction dans la colonne de rectification de réactif, et l'extraction de l'azéotrope de carbonate de diméthyle et d'éthanol via le port d'extraction de flux de phase gazeuse d'une partie supérieure de la colonne de rectification de réactif, et l'entrée d'une partie du mélange I comprenant du carbonate de méthyle d'éthyle, du carbonate de diéthyle, du carbonate de diméthyle et de l'éthanol dans le rebouilleur de colonne de rectification de réactif et l'entrée de l'autre partie du mélange I dans un port d'alimentation de la colonne d'élimination ;
(2) l'extraction d'une vapeur mixte de carbonate de méthyle d'éthyle et de carbonate de diméthyle à partir d'un port d'extraction de flux de phase gazeuse de la partie supérieure de la colonne d'élimination qui est ensuite soumise à une compression par le premier compresseur et est passée dans le rebouilleur de colonne de rectification de réactif pour fournir de la chaleur au rebouilleur de colonne de rectification de réactif, dans lequel une première partie de la matière entrant dans le rebouilleur de colonne de rectification de réactif est renvoyée vers un fond de la colonne de rectification de réactif, une deuxième partie de celle-ci est passée dans un port de reflux de la partie supérieure de la colonne d'élimination, et la partie restante de celle-ci est passée dans un port d'alimentation de la colonne de rectification de produit ;
(3) l'extraction d'une vapeur mixte de carbonate de méthyle d'éthyle et de carbonate de diméthyle à partir d'un port d'extraction de flux de phase gazeuse de la partie supérieure de la colonne de rectification de produit qui est ensuite soumise à une compression par le deuxième compresseur et est passée dans le rebouilleur de colonne de rectification de produit pour fournir de la chaleur au rebouilleur de colonne de rectification de produit, dans lequel une partie d'un mélange II comprenant du carbonate de diéthyle et du carbonate de méthyle d'éthyle est passée dans le rebouilleur de colonne de rectification de produit à partir d'un fond de la colonne de rectification de produit et l'autre partie est passée dans le port d'alimentation de la colonne d'élimination ; une première partie de la matière entrant dans le rebouilleur de colonne de rectification de produit est renvoyée vers le fond de la colonne de rectification de produit, une deuxième partie de celle-ci est renvoyée vers un port de reflux de la partie supérieure de la colonne de rectification de produit, et la partie restante de celle-ci comme matière première est passée dans la matière comprenant du carbonate de diméthyle et de l'éthanol ; et le carbonate de méthyle d'éthyle de qualité pour batterie peut être extrait à partir du milieu de la colonne de rectification de produit ;
l'azéotrope du carbonate de diméthyle et de l'éthanol est extrait du port d'extraction de flux de phase gazeuse de la partie supérieure de la colonne de rectification de réactif, puis entre dans un premier préchauffeur où l'azéotrope échange de la chaleur avec la matière comprenant du carbonate de diméthyle et de l'éthanol, et en outre une partie de l'azéotrope est passée dans un port de reflux de la partie supérieure de la colonne de rectification de réactif et l'autre partie est extraite et passée dans un système de séparation pour éliminer l'azéotrope ;
le procédé comprend le passage de la matière contenant du carbonate de diméthyle et de l'éthanol dans le mélangeur statique, puis le passage de la matière à travers le premier préchauffeur, un deuxième préchauffeur et un troisième préchauffeur en séquence, et l'entrée de la matière dans le pré-réacteur rempli de catalyseur pour effectuer une pré-réaction dans le pré-réacteur suivie par l'entrée de la matière résultante dans le port d'alimentation de la colonne de rectification de réactif.

2. Procédé selon la revendication 1, dans lequel le catalyseur est choisi parmi les catalyseurs alcalins ;
le catalyseur comprend un support et un élément métallique, et l'élément métallique est supporté sur le support ; l'élément métallique est choisi parmi les métaux alcalins ; et le support est au moins l'un parmi l'oxyde d'aluminium, l'oxyde de silicium et le tamis moléculaire.

3. Procédé selon la revendication 1, dans lequel
lorsque le dispositif I est utilisé, après que la température de la matière dans le rebouilleur de colonne de rectification de réactif est ajustée par le premier refroidisseur d'eau, une partie de celle-ci reflue vers le port de reflux de la colonne d'élimination, et l'autre partie de celle-ci est passée dans le port d'alimentation de la colonne de rectification de produit ;
lorsque le dispositif II est utilisé, après que la température de la matière dans le rebouilleur de colonne de rectification de produit est ajustée par le deuxième refroidisseur d'eau, une partie de celle-ci reflue vers le port de reflux de la colonne de rectification de produit et l'autre partie comme matière première est passée dans la matière contenant du carbonate de diméthyle et de l'éthanol.

4. Procédé selon la revendication 1, dans lequel le carbonate de méthyle d'éthyle de qualité pour batterie extrait du milieu de la colonne de rectification de produit est passé dans le deuxième préchauffeur pour le refroidissement puis extrait comme produit cible.

5. Procédé selon la revendication 1, dans lequel une partie de la matière comprenant du carbonate de diéthyle et du carbonate de méthyle d'éthyle extrait d'un fond de la colonne d'élimination est passée dans le rebouilleur de colonne d'élimination, et l'autre partie est passée dans un système de rectification pour éliminer le carbonate de diéthyle,
une partie de la matière entrant dans le rebouilleur de colonne d'élimination est renvoyée vers le fond de la colonne d'élimination, et l'autre partie est passée dans le troisième préchauffeur ; et
le rebouilleur de colonne d'élimination est alimenté en chaleur par de la vapeur.

6. Procédé selon la revendication 1, dans lequel les conditions opératoires de procédé de la colonne de rectification de réactif sont comme suit : la température de la partie supérieure de la colonne de rectification de réactif va de 60 à 70°C, une pression du fond de la colonne de rectification de réactif va de 100 à 150 KPa, une température du fond de la colonne de rectification de réactif va de 80 à 120°C, une pression du fond de la colonne de rectification de réactif va de 110 à 200 KPa et un taux de reflux de la partie supérieure de la colonne de rectification de réactif va de 2:1 à 8:1 ;
les conditions opératoires de procédé de la colonne d'élimination sont comme suit : une température de la partie supérieure de la colonne d'élimination va de 80 à 120°C, une pression de la partie supérieure de la colonne d'élimination va de 100 à 150 KPa, une température du fond de la colonne d'élimination va de 120 à 140°C, une pression du fond de la colonne d'élimination va de 120 à 200 KPa, et un rapport de reflux de la partie supérieure de la colonne d'élimination va de 1:1 à 6:1 ;
les conditions opératoires de procédé de la colonne de rectification de produit sont comme suit : une température de la partie supérieure de la colonne de rectification de produit va de 90 à 120°C, une pression de la partie supérieure de la colonne de rectification de produit va de 100 à 150 KPa, une température du fond de la colonne de rectification de produit va de 100 à 130°C, une pression du fond de la colonne de rectification de produit va de 120 à 200 KPa, et un rapport de reflux de la partie supérieure de la colonne de rectification de produit va de 2:1 à 8:1 ; et
la température de réaction du pré-réacteur va 90 à 120°C.

7. Procédé selon la revendication 1, dans lequel dans la matière comprenant du carbonate de diméthyle et de l'éthanol, un rapport molaire du carbonate de diméthyle par rapport à l'éthanol va de 0,5:1 à 2:1 ;
un taux de compression du premier compresseur va de 2 à 3, une pression de sortie du premier compresseur va de 200 à 300 KPa, et une température de sortie du premier compresseur va de 120 à 145°C ; et un taux de compression du deuxième compresseur va de 2,0 à 3,5 ; une pression de sortie du deuxième compresseur va de 200 à 360 KPa et une température de sortie du deuxième compresseur va de 125 à 145°C ;
dans la colonne de rectification de produit, le débit du port d'extraction de flux de phase gazeuse de la partie supérieure de la colonne de rectification de produit est de 1/2 à 3/4 du débit du port d'alimentation de la colonne de rectification de produit.
